# EUROPEAN PATENT SPECIFICATION

(11) **EP 0 574 618 B1**
(45) Date of publication and mention of the grant of the patent: **16.10.1996**
(21) Application number: 92201801.5
(22) Date of filing: 19.06.1992
(51) Int. Cl.: B41M 5/38, C09B 23/10, C07D 401/04

(54) **Thermally transferable fluorescent compounds**
Thermisch übertragbare fluoreszierende Verbindungen
Composés fluorescents pour transfert thermique

(43) Date of publication of application: 22.12.1993
(73) Proprietor: AGFA-GEVAERT naamloze vennootschap, B-2640 Mortsel (BE)
(72) Inventor: Vanmaele,Luc, DIE 3800,Septestraat 27 B 2640 Mortsel (BE); Loccufier,Johan, Septestraat 27 B 2640 Mortsel (BE)

(56) References cited:
- EP-A- 0 200 322

## Description

### 1. Field of the invention.

The present invention relates to fluorescent donor elements for use in a thermal transfer process.

### 2. Background of the invention.

In recent years, thermal transfer systems have been developed to obtain prints from pictures which have been generated electronically from a colour video camera. According to one way of obtaining such prints, an electronic picture is first subjected to colour separation by colour filters. The respective colour-separated images are then converted into electrical signals. These signals are then operated on to produce cyan, magenta and yellow electrical signals. These signals are then transmitted to a thermal printer. To obtain the print, a cyan, magenta or yellow dye-donor element is placed face-to-face with a dye-receiving element. The two are then inserted between a thermal printing head and a platen roller. A line-type thermal printing head is used to apply heat from the back of the dye-donor sheet. The thermal printing head has many heating elements and is heated up sequentially in response to the cyan, magenta and yellow signals. The process is then repeated for the other two colours. A colour hard copy is thus obtained which corresponds to the original picture viewed on a screen. This printing process is generally known under the name thermal dye sublimation transfer or thermal dye diffusion transfer. Further details of this process and an apparatus for carrying it out are contained in US 4621271.

The system described above has been used to obtain visible dye images. However, for security purposes, to inhibit forgeries or duplication, or to encode confidential information, it would be advantageous to create non-visual ultraviolet absorbing images that fluoresce with visible emission when illuminated with ultraviolet light.

It has been known to use fluorescent compounds in a continuous tone thermal transfer process. These fluorescent compounds are contained in the donor element and transfer or diffuse by themselves from this donor element to a receiving element. Such fluorescent compound containing donor elements are described in, for example, US 4871714, US 4876237, US 4876234, US 4866025, US 4866027, US 4891351, US 4891352 and US 5006503.

EP-A-200322 discloses indole derivatives having an optionally substituted phenyl in the 1-position and a hydrogenated pyrioline or pyrazine ring in the 3-position. The compounds are said to be pharmaceutically active.

### 3. Summary of the invention.

It is an object of the present invention to provide a new class of fluorescent compounds for use in a thermal transfer process.

In accordance with the present invention a donor element for use in a thermal dye diffusion transfer process is provided, said donor element comprising a support having on one side thereof a thermally transferable fluorescent heterocyclic compound dispersed in a polymeric binder, characterized in that said fluorescent compound corresponds to the following general formula I 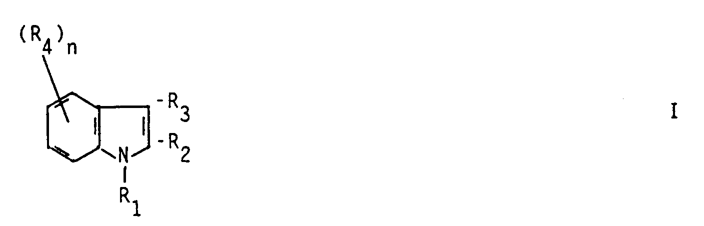 wherein:
R₁ and R₂ (same or different) represent hydrogen, a substituted or unsubstituted alkyl group or a substituted or unsubstituted aryl group;
R₃ represents a substituted or unsubstituted heterocyclic ring;
R₄ represents a substituent; and
n represents an integer from 0 to 4.

### 4. Detailed description of the invention.

Fluorescent compounds within the scope of said formula I that are suitable for use according to the present invention are compounds wich are very soluble in environmentally fully accepted organic solvents, such as 2-butanone, ethyl acetate, ethanol.

Examples of R₁ and R₂ include hydrogen, methyl, ethyl, propyl, isopropyl, butyl, isobutyl, 2-butyl, t-butyl, pentyl, hexadecyl, 2-methoxyethyl and phenyl.

Examples of R₃ include substituted or unsubstituted pyridine, substituted or unsubstituted isoquinoline, substituted or unsubstituted quinoline.

Examples of R₄ include hydrogen, halogen, nitro, alkoxy, thioalkoxy, nitrilo, carbonamido, sulfonamido, acylamino. Preferably n represents 0 or 1.

Fluorescent compounds according to the above general formula I which are particularly useful in the process of the present invention are listed in the following tables 1, 2, 3 and 4.

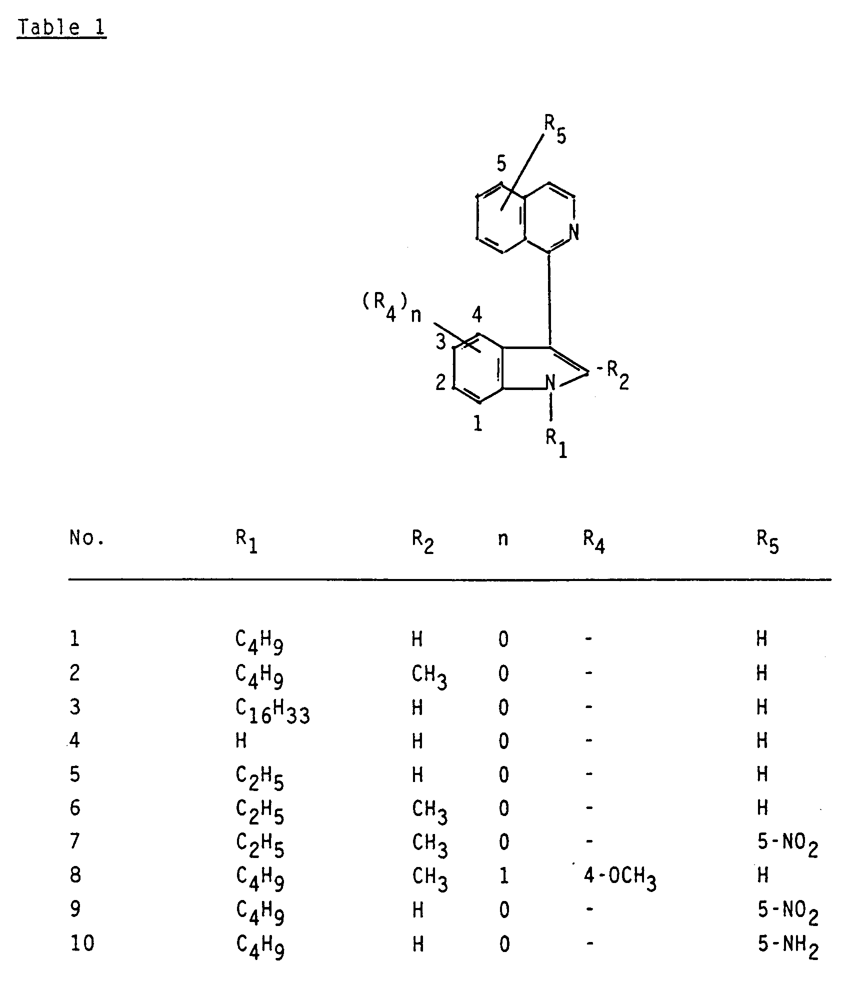

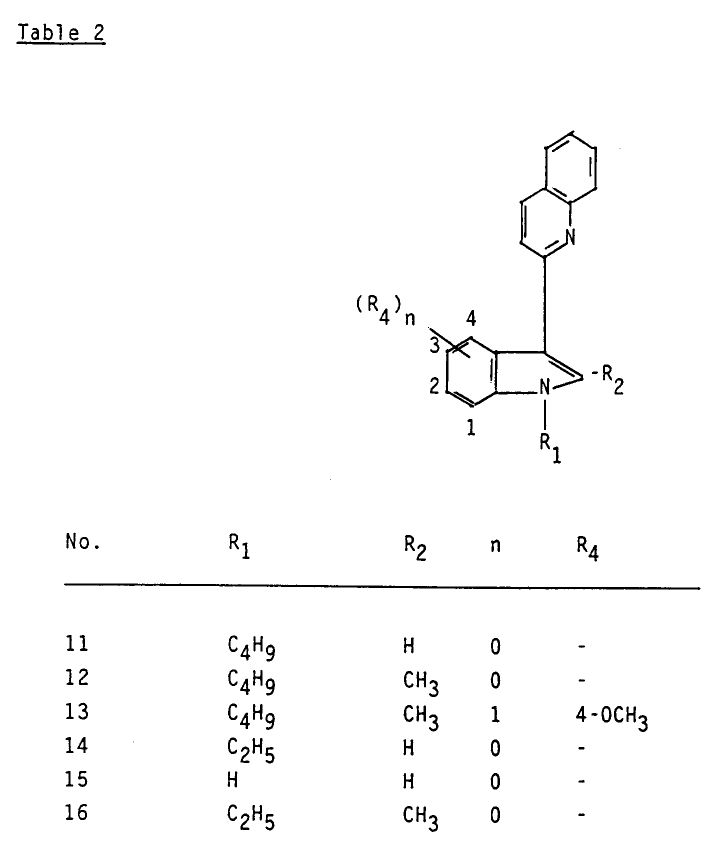

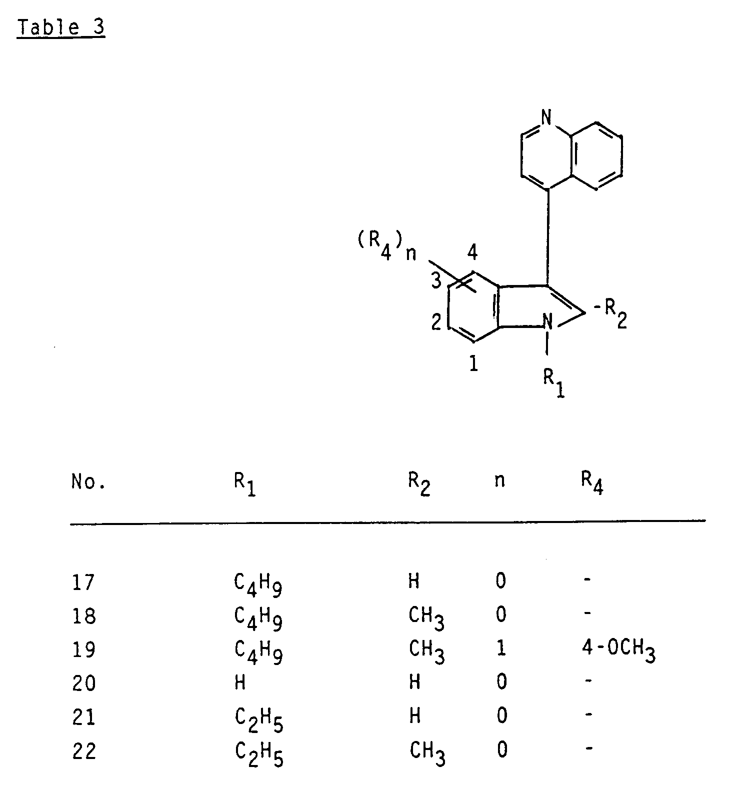

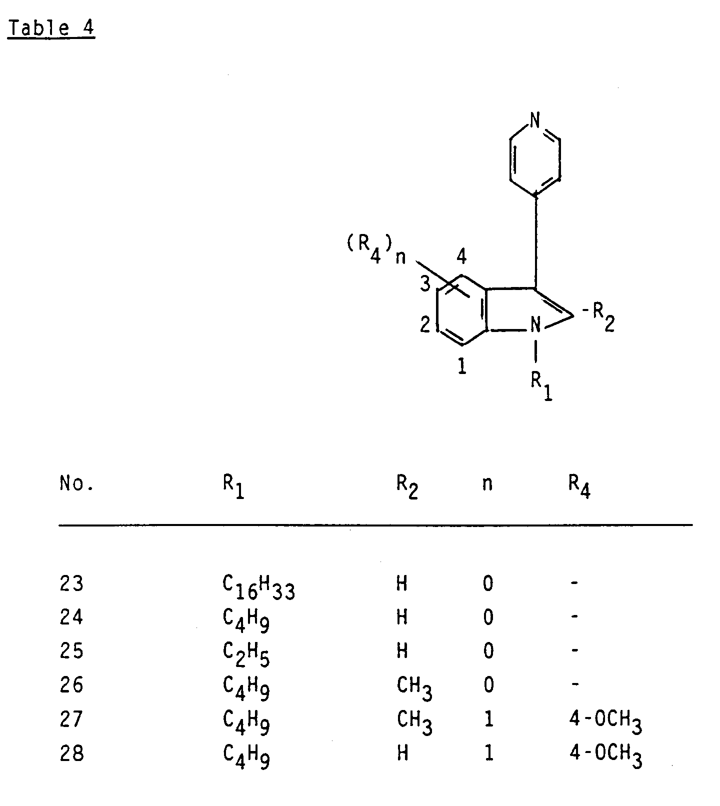

The fluorescent compounds according to the present invention are used in the donor element at a coverage of from about 0.01 to about 0.5 g/m².

A visible dye can also be used in a separate area of the donor element of the present invention provided it is transferable to the dye-receiving layer by the action of heat. Especially good results have been obtained with sublimable dyes. Examples of sublimable dyes include anthraquinone dyes, azo dyes, direct dyes, acid dyes, basic dyes and dyes which are disclosed in EP 432829, EP 432314, EP 400706, EP 485665 and EP 453020. Said dyes may be employed singly or in combination to obtain a monochrome. The dyes may be used at a coverage of from about 0.05 to about 1 g/m² and are preferably hydrophobic.

The fluorescent compound in the donor element of the invention is dispersed in a polymeric binder such as a cellulose derivative, e.g., cellulose acetate hydrogen phthalate, cellulose acetate, cellulose acetate propionate, cellulose acetate butyrate, cellulose triacetate; a polycarbonate; poly(styrene-co-acrylonitrile); a poly(sulfone) or a poly(phenylene oxide). The binder may be used at a coverage of from about 0.1 to about 5 g/m².

The fluorescent compound layer of the donor element may be coated on the support or printed thereon by a printing technique such as a gravure process.

Any material can be used as the support for the donor element of the invention provided it is dimensionally stable and can withstand the heat of the thermal printing heads. Such materials include polyesters such as poly(ethylene terephthalate); polyamides; polycarbonates; glassine paper; condenser paper; cellulose esters such as cellulose acetate; fluorine polymers such as polyvinylidene fluoride or poly(tetrafluoroethylene-cohexafluoropropylene); polyethers such as polyoxymethylene; polyacetals; polyolefins such as polystyrene, polyethylene, polypropylene or methylpentane polymers; and polyimides such as polyimide-amides and polyether-imides. The support generally has a thickness of from about 2 to about 30 µm. It may also be coated with a subbing layer, if desired.

The reverse side of the donor element is coated with a slipping layer to prevent the printing head from sticking to the donor element. Such a slipping layer would comprise a lubricating material such as a surface active agent, a liquid lubricant, a solid lubricant or mixtures thereof, with or without a polymeric binder. Preferred lubricating materials include oils or semi-crystalline organic solids that melt below 100°C such as poly(vinyl stearate), beeswax, perfluorinated alkyl ester polyethers, poly(caprolactone), silicone oil, poly(tetrafluoroethylene), carbowax, poly(ethylene glycols), or any of those materials disclosed in US 4717711, US 4737485, US 4738950, and US 4717712. Suitable polymeric binders for the slipping layer include poly(vinyl alcohol-co-butyral), poly(vinyl alcohol-co-acetal), poly(styrene), poly(vinyl acetate), cellulose acetate butyrate, cellulose acetate propionate, cellulose acetate or ethyl cellulose.

The amount of the lubricating material to be used in the slipping layer depends largely on the type of lubricating material, but is generally in the range of about 0.001 to about 2 g/m². If a polymeric binder is employed, the lubricating material is present in the range of 0.1 to 50 weight %, preferably 0.5 to 40, of the polymeric binder employed.

The receiving element that is used with the donor element of the invention usually comprises a support having thereon an image-receiving layer. The support may be a transparent film such as a poly(ether sulfone), a polyimide, a cellulose ester such as cellulose acetate, a poly(vinyl alcohol-co-acetal) or a poly(ethylene terephthalate). The support for the receiving element may also be reflective such as baryta-coated paper, polyethylene-coated paper, white polyester (polyester with white pigment incorporated therein), an ivory paper, a condenser paper or a synthetic paper such as duPont Tyvek.

The image-receiving layer may comprise, for example, a polycarbonate, a polyurethane, a polyester, polyvinyl chloride, poly(styrene-co-acrylonitrile), poly(caprolactone) or mixtures thereof. The image-receiving layer may be present in any amount which is effective for the intended purpose. In general, good results have been obtained at a concentration of from about 1 to about 5 g/m².

As noted above, the donor elements of the invention are used to form a transfer image. Such a process comprises imagewise-heating a donor element as described above and transferring a fluorescent compound image to a receiving element to form the transfer image.

The donor element of the invention may be used in sheet form or in a continuous roll or ribbon. If a continuous roll or ribbon is employed, it may have only the fluorescent compound thereon as described above or may have alternating areas of different dyes, such as sublimable magenta and/or yellow and/or cyan and/or black or other dyes. Thus, one-, two-, three- of four-colour elements (or higher numbers also) are included within the scope of the invention.

In a preferred embodiment of the invention, the donor element comprises a poly(ethylene terephthalate) support coated with sequential repeating areas of magenta, yellow, and cyan dye and the fluorescent material as described above, and the above process steps are sequentially performed for each colour to obtain a three-colour dye transfer image containing a fluorescent image.

Thermal printing heads which can be used to transfer fluorescent compound and dye from the donor elements of the invention are available commercially. There can be employed, for example, a Fujitsu Thermal Head (FTP-040 MCS001), a TDK Thermal Head F415 HH7-1089 or a Rohm Thermal Head KE 2008-F3.

A thermal transfer assemblage of the invention comprises (a) a donor element as described above, and (b) a receiving element as described above, the receiving element being in a superposed relationship with the donor element so that the fluorescent material layer of the donor element is in contact with the image receiving layer of the receiving element.

The following examples are provided to illustrate the invention in more detail without limiting, however, the scope thereof.

### Example 1 : Synthesis of compound 1

Compound 1 was prepared according to scheme 1.

### Scheme 1

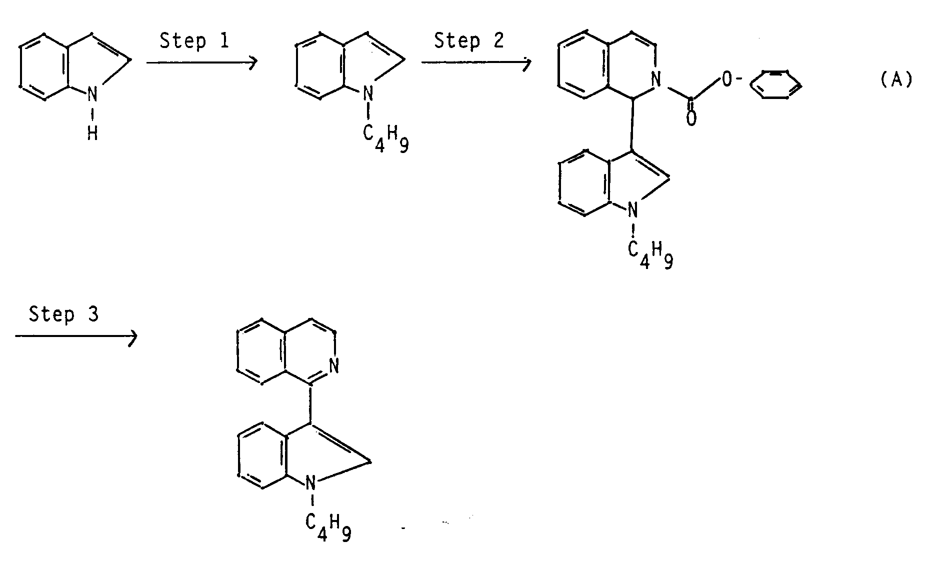

### Step 1

A solution of 28.7 g (0.25 mole) indole in 50 ml dimethylacetamide was added dropwise to a suspension of 10 g NaH (60 % in mineral oil, 0.25 mole). The temperature raised to 37°C. After the addition was completed, stirring was continued for 30 minutes. After 30 minutes, 27 ml (34 g, 0.25 mole) bromobutane was added over a period of 30 minutes. The reaction mixture was allowed to stand over night. The mixture was poured into 2 l water and extracted 3 times with 250 ml hexane. The pooled hexane extracts were dried over MgSO₄ and evaporated. The crude product (1-butyl-indole) was sufficiently pure for further use. (43 g (99 %)).

### Step 2

4.33 g (0.25 mole) 1-butyl-indole was dissolved in 25 ml toluene. 8.06 ml (8.86 g, 0.069 mole) isoquinoline was added and the reaction mixture was cooled in an ice bath. 3.39 ml (4.23 g, 0.027 mole) phenyl chloroformate was added dropwise at 0°C. The reaction was allowed to continue for 1 hour. The precipitated isoquinoline.HCl was removed by filtration and the solvent was evaporated under reduced pressure. The residue was dissolved in 200 ml methyl-t.butyl ether and extracted with 100 ml 1 N HCl and washed with 100 ml water. The organic layer was dried over MgSO₄ and evaporated under reduced pressure. The oily residue (compound A) was used without further purification (7.36 g (70 %)).

### Step 3

7.36 g (0.0174 mole) of compound A was dissolved in 35 ml of tetrahydrofurane. 5.94 g (0.026 mole) 2,3-dichloro-5,6-dicyano-1,4-benzoquinone was added and the reaction was allowed to continue for 1 hour. 175 ml CH₂Cl₂ was added and the reaction mixture was extracted with 175 ml 0.2 N NaOH. The organic layer was washed with 100 ml 0.1 N NaOH, 100 ml of a 25 % NaCl-solution, 100 ml water and dried over MgSO₄. The solvent was removed under reduced pressure and the product was isolated by preparative column chromatography (eluent : hexane/ethylacetate 2/1; Rf = 0.25). 4.6 g (88 %) of compound 1 were obtained.

### Example 2 : Synthesis of compound 4

Compound 4 was prepared according to Scheme 2.

### Scheme 2

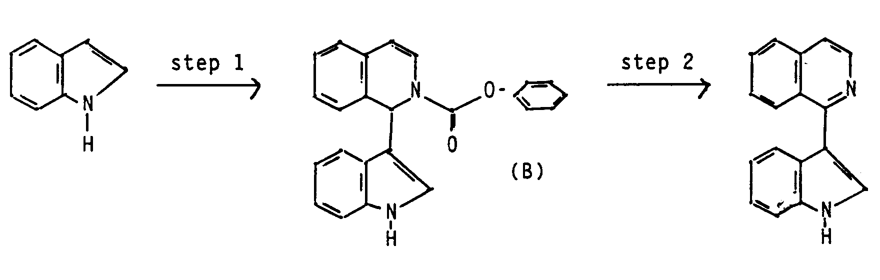

### Step 1

5.85 g (0.05 mole) indole and 16.3 ml (17.9 g, 0.139 mole) isoquinoline were dissolved in 20 ml toluene. The solution was cooled in an ice bath. A solution of 9.77 ml (12.2 g, 0.078 mole) phenyl chloroformate in 20 ml toluene was added over a period of 1 hour at 0°C. 50 ml methyl-t.butyl ether was added, the precipitated product was isolated by filtration and washed with methyl-t.butyl ether. The isolated product was dissolved in 100 ml methanol on heating. 50 ml water was added and the mixture was allowed to stand for 15 minutes. The precipitated product was isolated by filtration, washed with methanol and dried under vacuum. 14.47 g (79 %) of compound B were obtained (m.p. = 144°C).

### Step 2

7.32 g (0.02 mole) of compound B was dissolved in 40 ml tetrahydrofurane. 6.81 g (0.03 mole) 2,3-dichloro-5,6-dicyano-1,4-benzoquinone was added. The reaction was allowed to continue for one and a half hour. 200 ml CH₂Cl₂ was added; the reaction mixture was extracted with 200 ml 0.2 N NaOH and a second time with 100 ml 0.1 N NaOH. Addition of a small portion methanol was required to dissolve a precipitated product. The organic layer was washed twice with 100 ml water and dried over MgSO₄. The solvent was evaporated under reduced pressure, the residue was treated with hot methyl-t.butyl ether and isolated by filtration. 3.57 g (73 %) of compound 4 were obtained (m.p. = 210°C).

### Example 3 : Synthesis of compound 2

Compound 2 was prepared according to scheme 3.

### Scheme 3

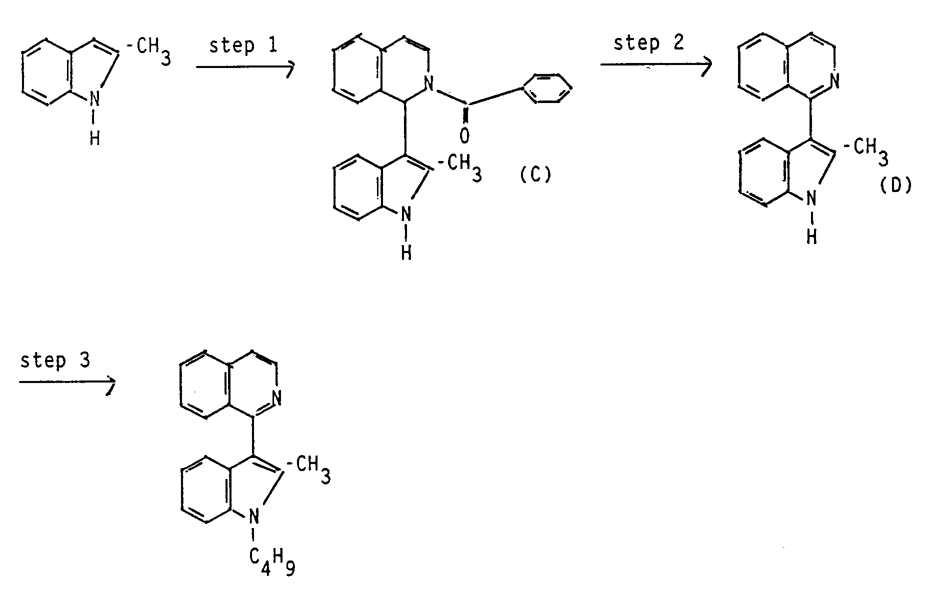

### Step 1

A solution of 5.85 ml (7.08 g, 0.05 mole) of benzoylchloride in 20 ml toluene was added dropwise to a solution of 16.3 ml (17.9 g, 0.139 mole) isoquinoline and 6.55 g (0.05 mole) 2-methyl-indole in 20 ml toluene. The reaction products precipitated from the reaction medium. After 1 hour 60 ml methanol was added and the mixture was stirred for 30 minutes. The product was isolated by filtration and recrystallized from methanol. 11.9 g (65 %) of compound C were obtained (m.p. = 210°C).

### Step 2

5.46 g (0.015 mole) of compound C was dissolved in 35 ml tetrahydrofurane. 6.81 g (0.03 mole) 2,3-dichloro-5,6-dicyano-1,4-benzoquinone was added and the reaction was allowed to continue for 1 hour. After 1 hour, 11 ml (0.08 mole) triethylamine was added and the product precipitated from the medium. The product was isolated by filtration, washed with tetrahydrofurane and dried under vacuum. The crude product (compound D) was used without further purification.

### Step 3

A suspension of 2.58 g (0.01 mole) of compound D in 20 ml dimethyl acetamide was added to a suspension of 0.4 g NaH (60 % in mineral oil, 0.01 mole) in 5 ml dimethylacetamide. The reaction mixture was heated till 50°C. The products dissolved. After cooling down till room temperature, 1.19 ml (1.52 g, 0.011 mole) 1-bromobutane was added dropwise. The reaction mixture was allowed to stand over night. The mixture was poured into 200 ml water and extracted twice with 100 ml methyl-t.butyl ether. The combined extracts were dried over MgSO₄. The solvent was removed under reduced pressure and the product was isolated by preparative column chromatography (eluent : CH₂Cl₂/MeOH 95/5; Rf = 0.44). On standing the product crystallized. It was recrystallized from methyl-t.butyl ether. 1 g (32 %) of compound 2 was obtained (m.p. = 94°C).

### Example 4 : Synthesis of compound 23

Compound 23 was prepared according to scheme 4.

### Scheme 4

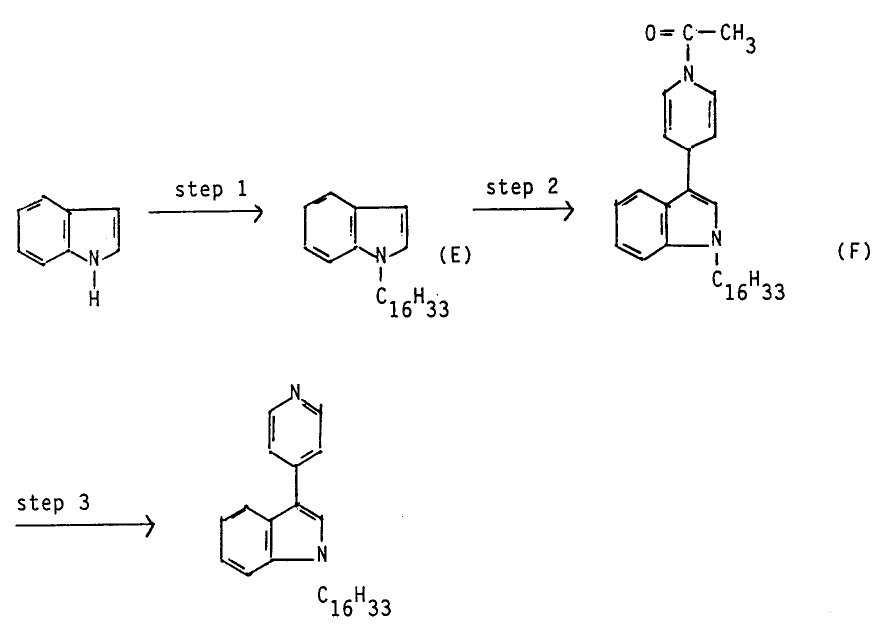

### Step 1

A solution of 28.7 g (0.25 mole) indole in 50 ml dimethylacetamide was added dropwise to a suspension of 10 g NaH (60 % in mineral oil, 0.25 mole) in 250 ml dimethylacetamide. After the addition was completed, stirring was continued for half an hour. After 30 minutes, 76.3 g (0.25 mole) cetyl bromide was added over a period of 30 minutes. The reaction was allowed to continue for 2 hours. The reaction mixture was poured into 2 l water and extracted 3 times with 400 ml hexane. The combined hexane extracts were washed with 400 ml of a 25 % NaCl-solution, dried over MgSO₄, and evaporated under reduced pressure. The residue was further purified by preparative column chromatography (eluent CH₂Cl₂/hexane 20/80; Rf = 0.23). 62 g of compound E were obtained (73 %).

### Steps 2 and 3

8.03 ml (8.88 g, 0.11 mole) acetyl chloride were dissolved in 75 ml acetonitrile. 17.8 ml (17.4 g, 0.22 mole) pyridine were added. The temperature was kept between 20 and 25°C. 35.9 g (0.1 mole) of compound E were added and the reaction mixture was heated till 45°C. The reaction was allowed to continue for 2 hours. After 2 hours, the reaction mixture was poured into 1.5 l water and extracted twice with 400 ml CH₂Cl₂. The combined extracts were washed with 200 ml 1N HCl, 200 ml of a 25 % NaCl-solution and 200 ml water and dried over MgSO₄. The solvent was removed under reduced pressure. A portion of the dihydropyridine (compound F) was oxidized by air and was isolated by preparative column chromatography (eluent : hexane/ethyl acetate 70/30; Rf = 0.1). The product was recrystallized from methanol. 0.54 g (1.4 %) of compound 23 were obtained (m.p. = 58°C).

### Example 5 : Synthesis of compound 11 and compound 17

Compound 11 and compound 17 were prepared according to scheme 5.

### Scheme 5

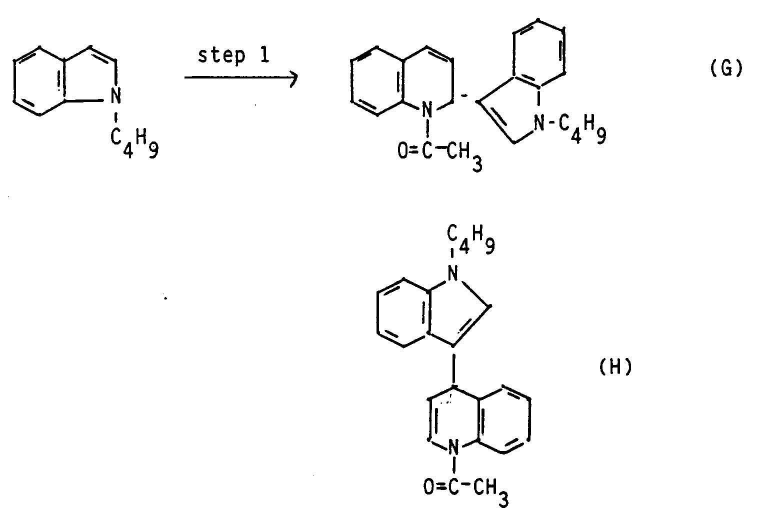

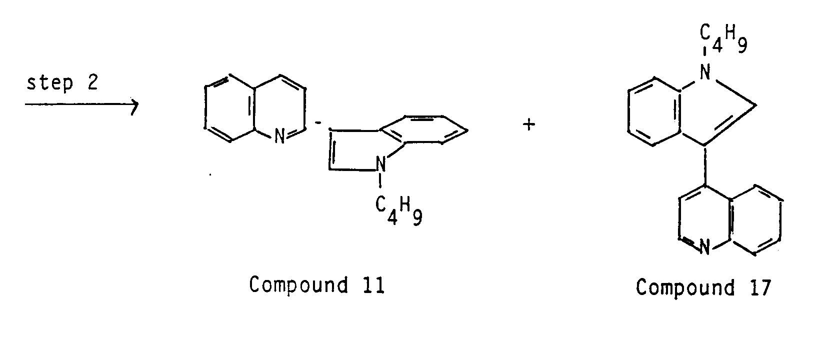

### Step 1

27.1 ml (29.6 g, 0.23 mole) quinoline were added slowly to a solution of 8.03 ml (8.87 g, 0.11 mole) acetyl chloride in 75 ml acetonitrile. The temperature was kept between 10 and 15°C. 17.3 g (0.1 mole) 1-butyl-indole (see example 1) was added slowly. The reaction mixture was heated till 50°C. The reaction was allowed to continue for 1 hour. After 1 hour, the mixture was poured into 1 l water and extracted twice with 250 ml methyl-t.butyl ether. The pooled extracts were washed twice with 100 ml 1N HCl and once with 100 ml water. The organic layer was dried over MgSO₄ and the solvent was removed under reduced pressure. The two isomers were separated by preparative column chromatography (eluent : CH₂Cl₂/ethylacetate 99/1; Rf compound H = 0.41; Rf compound G = 0.35). Compound G crystallized on standing and was treated with hexane, isolated by filtration and washed with methyl-t.butyl-ether. Compound G : 14.2 g (41 %) (m.p. = 89°C). Compound H : 8.8 g (25 %).

### Step 2

6.88 g (0.02 mole) of compound G were dissolved in 50 ml tetrahydrofurane. 6.81 g (0.03 mole) 2,3-dichloro-5,6-dicyano-1,4-benzoquinone was added. After 1 hour, the oxidation was incomplete and 2.3 g (0.01 mole) 2,3-dichloro-5,6-dicyano-1,4-benzoquinone in 25 ml tetrahydrofurane was added. A second addition of 2.3 g (0.01 mole) in 25 ml CH₂Cl₂ was required. 200 ml CH₂Cl₂ was added and the reaction mixture was extracted with 200 ml 0.5 N NaOH. The organic layer was washed twice with 100 ml water and dried over MgSO₄. The solvent was removed under reduced pressure and the product was isolated by preparative column chromatography (eluent : hexane / ethylacetate 85/15; Rf = 0.4). 3.3 g (55 %) of compound 11 were obtained.

8.4 g of compound H were dissolved in 25 ml tetrahydrofuran. 8.06 g (0.036 mole) 2,3-dichloro-5,6-dicyano-1,4-benzoquinone was added and the reaction was allowed to continue for 1 hour. 125 ml CH₂Cl₂ was added and the reaction mixture was extracted with 125 ml 1N NaOH. The organic layer was washed twice with 100 ml water and dried over MgSO₄. The solvent was removed under reduced pressure and the product was isolated by preparative column chromatography (eluent : hexane/ethylacetate 1/1; Rf = 0.55). 4.5 g (63 %) of compound 17 were obtained.

### Example 6

A donor element for use in a thermal transfer process was prepared as follows.

To avoid sticking of the donor element to the thermal printing head the rear side of a 5 µm polyethylene terephthalate support was provided first with a solution for forming a slipping layer, said solution comprising 10 g of co(styrene/acrylonitrile) comprising 104 styrene units and 53 acrylonitrile units, which copolymer is sold under the trade mark LURAN 378 P by BASF AG, D-6700 Ludwigshafen, West Germany, 10 g of a 1% solution of polysiloxane polyether copolymer sold under the trade mark TEGOGLIDE 410 by Th. Goldschmidt AG, D-4300 Essen 1, Goldschmidtstrasse 100, West Germany, and sufficient ethyl methyl ketone solvent to adjust the weight of the solution to a total of 100 g. From this solution a layer having a wet thickness of 15 µm was printed by means of a gravure press. The resulting layer was dried by evaporation of the solvent.

A solution of 50 mg of fluorescent compound, the nature of which is identified in Table 5 below, and 50 mg of co-acrylonitrile-styrene binder in 10 ml of methylethylketone as solvent was prepared. From this solution a layer having a wet thickness of 100 µm was coated on 5 µm polyethylene terephthalate film. The resulting layer was dried by evaporation of the solvent.

A commercially available material supplied by MITSUBISHI, type CK 100 S was used as receiving element.

The dye-donor element was printed in combination with the receiving element in a color video printer supplied by MITSUBISHI, type CP 100 E.

The receiver sheet was separated from the dye-donor element and the relative emission was visually evaluated using a fixed intensity 366 nm excitation beam (CAMAG UV-Cabinet II) and a fixed intensity 254 nm beam.

**TABLE 5**

| Fluorescent compound no. | Visual colour (at 254 nm) | Intensity | Visual colour (at 366 nm) | Intensity |
|---|---|---|---|---|
| 1 | yellowish-green | s | yellowish-white | vs |
| 2 | yellowish-green | s | yellowish-white | vs |
| 3 | yellow | s | yellowish | vs |
| 4 | yellowish | vw | yellowish-brown | w |
| 11 | yellowish | s | white | vs |
| 17 | yellow | es | yellow | es |
| 23 | yellowish-white | s | white | vs |
| vw = very weak; w = weak; s = strong; vs = very strong; es = extremely strong | | | | |

## Claims

1. Donor element for use in a thermal dye diffusion transfer process comprising a support having on one side thereof a thermally transferable fluorescent heterocyclic compound dispersed in a polymeric binder, characterized in that said fluorescent compound corresponds to the following general formula I 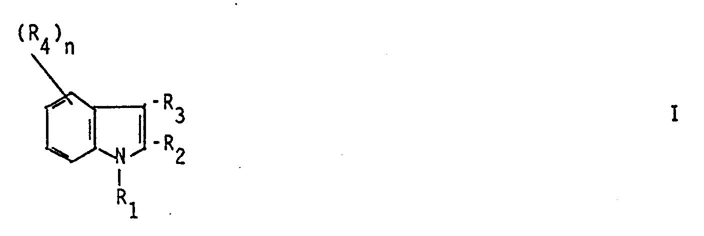 wherein:
R₁ and R₂ (same or different) represent hydrogen, a substituted or unsubstituted alkyl group or a substituted or unsubstituted aryl group;
R₃ represents a substituted or unsubstituted heterocyclic ring;
R₄ represents a substituent; and
n represents an integer from 0 to 4.

2. Donor element according to claim 1, wherein R₃ represent quinoline or isoquinoline or pyridine, said rings being substituted or unsubstituted.

3. Donor element according to claim 1 or 2, wherein R₁ represents an alkyl group, R₂ represents hydrogen or an alkyl group and n represents 0.

4. Donor element according to any one of the preceding claims, wherein said polymeric binder is co(styrene-acrylonitrile).

5. Donor element according to any one of the preceding claims, wherein said fluorescent compound is present at a coverage of from 0.01 to 0.5 g/m².

6. Donor element according to any one of the preceding claims, wherein the other side of the support is provided with a slipping layer comprising a lubricant.

7. Donor element according to any one of the preceding claims, wherein said donor element comprises sequential repeating areas of magenta, yellow and cyan dye, and said fluorescent compound.

8. A fluorescent compound as defined in any of claims 1 to 3 with the proviso that compounds wherein R₁ represents phenyl optionally substituted with halogen, lower alkyl or trifluoro-methyl, or a hetero aromatic group are excluded when R₃ represents a heterocyclic ring according to the following formula: 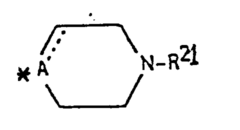
"A" is nitrogen or carbon, and the dotted line indicates - when A is carbon - an optional bond; "*" denotes the linking position;
R²¹ is hydrogen, cycloalkyl, lower alkyl or lower alkenyl, optionally substituted with one or two hydroxy groups, any hydroxy group present being optionally esterified with an aliphatic carboxylic acid radical having from two to twenty-four carbon atoms inclusive,
or R²¹ is the group 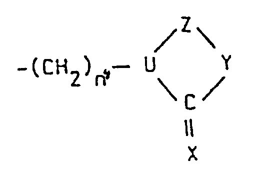 wherein "n'" is an integer of 2-6;
X is oxygen or sulfur, or > C = X may constitute the group \CH = when Y is =N- or =CH-;
Y is oxygen, sulfur, CH₂ or N R³¹, where R³¹ is hydrogen or lower alkyl, lower alkenyl or a cycloalkylmethyl group, said "cycloalkyl" having from three to six carbon atoms inclusive;
Z is -(CH₂)ₘ -, "m" being 2 or 3, or Z is -CH=CH- or 1,2-phenylene optionally substituted with halogen or trifluoromethyl, or Z is -CO(or S)CH₂-;
U is nitrogen or carbon.

## Patentansprüche

1. Donorelement zur Anwendung in einem Thermodiffusionsübertragungsverfahren, das einen Träger enthält, auf dessen eine Seite sich eine thermisch übertragbare, fluoreszierende, heterocyclische, in ein polymeres Bindemittel dispergierte Verbindung befindet, dadurch gekennzeichnet, daß die fluoreszierende Verbindung der folgenden allgemeinen Formel I entspricht : 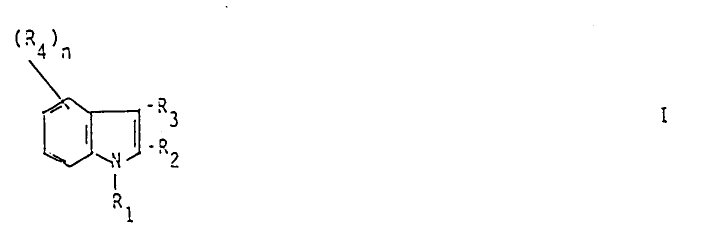 in der bedeuten :
R₁ und R₂ (gleich oder verschieden) Wasserstoff, eine substituierte oder nicht-substituierte Alkylgruppe oder eine substituierte oder nicht-substituierte Arylgruppe,
R₃ einen substituierten oder nicht-substituierten, heterocyclischen Ring,
R₄ einen Substituenten, und
n eine ganze Zahl zwischen 0 und 4.

2. Donorelement nach Anspruch 1, dadurch gekennzeichnet, daß R₃ einen substituierten oder nicht-substituierten Chinolin-, Isochinolin- oder Pyridinring bedeutet.

3. Donorelement nach Anspruch 1 oder 2, dadurch gekennzeichnet, daß R₁ eine Alkylgruppe, R₂ Wasserstoff oder eine Alkylgruppe und n 0 bedeutet.

4. Donorelement nach irgendeinem der vorstehenden Ansprüche, dadurch gekennzeichnet, daß das polymere Bindemittel Co(styrol-acrylonitril) ist.

5. Donorelement nach irgendeinem der vorstehenden Ansprüche, dadurch gekennzeichnet, daß die fluoreszierende Verbindung in einem Verhältnis zwischen 0,01 und 0,5 g/m² enthalten ist.

6. Donorelement nach irgendeinem der vorstehenden Ansprüche, dadurch gekennzeichnet, daß die andere Trägerseite mit einer ein Schmiermittel enthaltenden Gleitschicht überzogen ist.

7. Donorelement nach irgendeinem der vorstehenden Ansprüche, dadurch gekennzeichnet, daß das Donorelement sich sequentiell wiederholende Bereiche mit Magenta-, Gelb- und Cyanfarbstoff und die fluoreszierende Verbindung enthält.

8. Fluoreszierende Verbindung wie in irgendeinem der vorstehenden Ansprüche definiert, unter der Bedingung, daß Verbindungen, bei der R₁ eine gegebenenfalls mit Halogen, einer niedrigeren Alkylgruppe, einer Trifluormethylgruppe oder einer heterocyclischen aromatischen Gruppe substituierte Phenylgruppe darstellt, ausgeschlossen sind, falls R₃ einen heterocyclischen Ring nach der folgenden Formel darstellt : 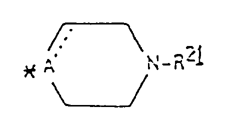 in der bedeuten :
"A" Stickstoff oder Kohlenstoff und die punktierte Linie - falls A Kohlenstoff ist - eine gegebenenfalls zusätzliche Verbindung
"*" die Stellung der Verbindung,
R²¹ Wasserstoff, eine Cycloalkyl-, eine niedrigere Alkyl- oder eine niedrigere Alkenylgruppe, die gegebenenfalls mit einer oder zwei Hydroxylgruppen substituiert ist, wobei jede enthaltene Hydroxylgruppe gegebenenfalls mit einem aliphatischen Carbonsäureradikal mit zwei bis zwanzig Kohlenstoffatomen verestert ist,
oder R₂₁ die Gruppe 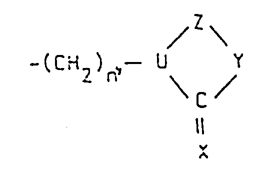 in der bedeuten :
n eine ganze Zahl zwischen 2 und 6,
X Sauerstoff oder Schwefel, oder >C = X die Gruppe \CH= falls Y =H- oder =CH- ist,
Y Sauerstoff, Schwefel, CH₂ oder N R³¹, wobei R³¹ Wasserstoff, eine niedrigere Alkylgruppe, eine niedrigere Alkenylgruppe oder eine Cycloalkylmethylgruppe darstellt, wobei die "Cycloalkylgruppe" drei bis sechs Kohlenstoffatome enthält,
Z -(CH₂)ₘ- wobei "m" 2 oder 3 ist, oder Z -CH=CH- oder 1,2-Phenylen, das gegebenenfalls mit Halogen oder Trifluormethyl substituiert ist, oder Z -CO(oder S)CH₂-,
U Stickstoff oder Kohlenstoff.

## Revendications

1. Elément donneur à utiliser dans un procédé de transfert de colorant par thermodiffusion, comprenant un support sur lequel se trouve sur un côté un composé hétérocyclique fluorescent thermotransférable dispersé dans un liant polymère, caractérisé en ce que le composé fluorescent correspond à la formule générale suivante I 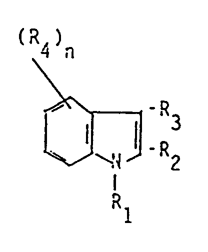 dans laquelle:
R₁ et R₂ ( identiques ou différents), représentent un atome d' hydrogène, un groupe alkyle substitué ou non substitué ou un groupe aryle substitué ou non substitué;
R₃ représente un noyau hétérocyclique substitué ou non substitué;
R₄ représente un substituant; et
n représente un nombre entier de 0 à 4.

2. Elément donneur selon la revendication 1, caractérisé en ce que R₃ représente la quinoline ou l'isoquinoline ou la pyridine, lesquels noyaux peuvent être substitués ou non substitués.

3. Elément donneur selon la revendication 1 ou 2, caractérisé en ce que R₁ représente un groupe alkyle, R₂ représente un atome d'hydrogène ou un groupe alkyle et n représente 0.

4. Elément donneur selon l'une quelconque des revendications précédentes, caractérisé en ce que le liant polymère est le poly(styrène-*co*-acrylonitrile).

5. Elément donneur selon l'une quelconque des revendications précédentes, caractérisé en ce que le composé fluorescent est présent à un taux d'application entre 0,01 et 0,5 g/m².

6. Elément donneur selon l'une quelconque des revendications précédentes, caractérisé en ce que l'autre côté du support est muni d'une couche de glissement comprenant un lubrifiant.

7. Elément donneur selon l'une quelconque des revendications précédentes, caractérisé en ce que l'élément donneur comprend des zones séquentielles répétitives de colorant magenta, jaune et cyan, et le composé fluorescent.

8. Un composé fluorescent tel que défini dans l'une quelconque des revendications 1 à 3, à condition que les composés dans lesquels R₁ représente un groupe phényle éventuellement substitué par un atome d'halogène, un groupe alkyle inférieur, un groupe trifluorométhyle ou un groupe hétéro aromatique, soient exclus quand R₃ représente un noyau hétérocyclique selon la formule suivante: 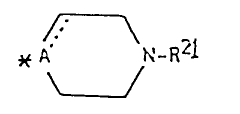 dans laquelle:
A représente un atome d'azote ou un atome de carbone, et les lignes pointillés indiquent - quand A est un atome de carbone - une liaison optionnelle;
"*" indique la position de liaison;
R²¹ représente un atome d'hydrogène, un groupe cycloalkyle, un groupe alkyle inférieur ou un groupe alcényle inférieur, éventuellement substitués avec un ou deux groupes hydroxy, n'importe quel groupe hydroxy présent étant éventuellement estérifié avec un radical d'acide carboxylique aliphatique ayant de deux jusqu'à vingt-quatre atomes de carbone y compris,
ou R²¹ est le groupe 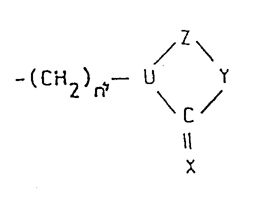 dans lequel
n est un nombre entier de 2 à 6;
X est un atome d'oxygène ou de soufre, ou > C = X peut constituer le groupe \CH = quand Y est =N- ou =CH-;
Y est un atome d'oxygène, un atome de soufre, CH₂ ou N R³¹, dans lequel R³¹ est un atome d'hydrogène ou un groupe alkyle inférieur, un groupe alcényle inférieur ou un groupe cycloalkylméthyle, lequel "cycloalkyle" ayant de trois à six atomes de carbone y compris;
Z représente -(CH₂)ₘ-, "m" étant 2 ou 3, ou Z représente -CH=CH- ou le 1,2-phénylène éventuellement substitué avec un atome d'halogène ou un groupe trifluorométhyle, ou Z représente -CO(ou S)CH₂-;
U représente un atome d'azote ou un atome de carbone.
